# EUROPÄISCHE PATENTANMELDUNG

(11) **EP 2 108 385 A1**
(43) Veröffentlichungstag der Anmeldung: **14.10.2009**
(21) Anmeldenummer: 09004320.9
(22) Anmeldetag: 26.03.2009
(51) Int. Cl.: A61L 27/34, A61L 27/54, A61L 29/08, A61L 29/16, A61L 31/10, A61L 31/16

(54) **Silberhaltige Polyurethanharnstofflösung**

(30) Priorität: 08.04.2008 EP 08154206
(71) Anmelder: Bayer MaterialScience AG, 51368 Leverkusen (DE)
(72) Erfinder: Köcher, Jürgen, Dr., 40764 Langenfeld (DE); Eiden, Stefanie, Dr., 51371 Leverkusen (DE)

(57) **Zusammenfassung**

Die vorliegende Erfindung betrifft Lösung eines nichtionischen Polyurethanharnstoffes, welche als antimikrobiellen Wirkstoff einen silberhaltigen Bestandteil aufweist.

## Beschreibung

Die vorliegende Erfindung betrifft eine Polyurethanharnstofflösung, welche einen antimikrobiellen silberhaltigen Bestandteil aufweist. Weiterer Gegenstand der vorliegenden Erfindung ist ein Verfahren zur Herstellung einer entsprechenden Polyurethanharnstofflösung sowie deren Verwendung.

Kunststoff- und Metallgegenstände werden im medizinischen Bereich sehr häufig verwendet. Beispiele für derartige Materialien sind Implantate, Kanülen oder Katheter. Problematisch bei der Verwendung dieser Produkte ist das leichte Besiedeln der Oberflächen dieser Materialien mit Keimen. Die Folge der Verwendung eines mit Bakterien besiedelten Gegenstandes, wie eines Implantates, einer Kanüle oder eines Katheters, sind oft Infektionen durch Bildung eines Biofilms. Besonders gravierend sind solche Infektionen im Bereich von zentralvenösen Kathetern sowie im urologischen Bereich, wo Katheter verwendet werden.

Es wurden in der Vergangenheit bisher zahlreiche Versuche unternommen, die Besiedlung von Oberflächen mit Bakterien und somit Infektionen zu verhindern. Häufig wurde versucht, die Oberfläche von medizinischen Implantaten oder Kathetern mit Antibiotika zu imprägnieren. Dabei muss allerdings mit der Bildung und Selektion von resistenten Bakterien gerechnet werden.

Ein weiterer Ansatz zur Verhinderung von Infektionen bei der Verwendung von Implantaten oder Kathetern ist die Verwendung von Metallen oder Metalllegierungen, z. B. bei Kathetern.

Von besonderer Bedeutung ist hierbei die antibakterielle Wirkung von Silber. Silber und Silbersalze sind schon seit vielen Jahren als antimikrobiell wirksame Substanzen bekannt. Die antimikrobielle Wirkung von Oberflächen, die Silber enthalten, beruht auf der Freisetzung von Silberionen. Der Vorteil von Silber besteht in seiner hohen Toxizität gegenüber Bakterien schon in sehr niedrigen Konzentrationen. Hardes et al., Biomaterials 28 (2007) 2869-2875, berichten von einer bakteriziden Aktivität von Silber bis hinunter zu einer Konzentration von 35 ppb. Demgegenüber ist Silber auch bei einer deutlich höheren Konzentration gegenüber Säugetierzellen noch nicht toxisch. Ein weiterer Vorteil ist die geringe Neigung der Bakterien zur Bildung von Resistenzen gegenüber Silber.

Verschiedene Ansätze zur Ausrüstung medizinischer Geräte mit Silber wie beispielsweise Kathetern sind in der Literatur beschrieben. Ein Ansatz ist die Verwendung von metallischem Silber auf Katheteroberflächen. Ein Katheter, der an der Außenwand eine versilberte Oberfläche enthält, wird in US 3800087 beschrieben. Nachteilig ist dabei, dass das Silber bei den Belastungen des Katheters schlecht haftet, z. B. bei Lagerung in Körperflüssigkeiten wie Urin, Reibung beim Einführen und Herausziehen aus dem Körper oder durch wiederholtes Biegen des Katheters. Eine Verbesserung der Haftung der Silberschicht auf einem Katheterkunststoff beschreibt DE 4328999, indem zwischen Kunststoff und Silberschicht besser haftende Metallschichten aufgebracht werden. Aufgebracht wird das Silber bei den beschriebenen Produkten durch Aufdampfen in einer Vakuumkammer, Sputtern oder auch durch lonenimplantation. Diese Verfahren sind sehr umständlich und kostspielig. Nachteilig ist auch, dass die Menge an aufgedampftem elementarem Silber relativ hoch ist, aber nur sehr geringe Mengen aktiver Silberionen an die umgebende Flüssigkeit abgegeben werden. Weiterhin kann man mit diesen Verfahren nur die Außenseite eines Implantates oder eines Katheters beschichten. Es ist aber bekannt, dass sich auch an der Innenseite eines Katheters leicht Bakterien anhaften, was zur Biofilmbildung und Infektion des Patienten führt.

Metallische Beschichtungen von medizinischen Geräten haben jedoch nicht nur den Nachteil der schlechten Haftung auf dem Kathetermaterial, sondern auch, dass ein Aufbringen auf den Innenseiten des Katheders nicht oder nur sehr aufwändig möglich ist.

Viele Anwendungen beschäftigen sich mit der Verwendung von Silbersalzen in antimikrobiellen Beschichtungen, die auf medizinische Implantate oder Katheter aufgebracht werden. Gegenüber metallischem Silber haben Silbersalze den Nachteil, dass in der imprägnierten Schicht neben dem wirksamen Silber noch Anionen enthalten sind, die unter Umständen toxisch sein können, wie beispielsweise Nitrat im Silbernitrat. Ein weiteres Problem ist die Freisetzungsrate von Silberionen aus Silbersalzen. Manche Silbersalze wie Silbernitrat sind sehr gut wasserlöslich und werden deshalb eventuell zu schnell aus der Oberflächenbeschichtung in das umliegende Medium abgegeben. Andere Silbersalze wie Silberchlorid sind so schwerlöslich, dass Silberionen unter Umständen zu langsam in die Flüssigkeit abgegeben werden.

Weitere Veröffentlichungen, wie beispielsweise die WO 2004/017738 A, die WO 2001/043788 A und die US 2004/0116551 A beschreiben ein Konzept, durch Kombination verschiedener Silbersalze zu einer silberhaltigen Beschichtung zu kommen, die kontinuierlich Silberionen freisetzt. Die verschiedenen Silbersalze werden mit unterschiedlichen Polymeren, beispielsweise Polyurethanen, gemischt, und die Kombination von Silbersalzen verschiedener Wasserlöslichkeit ist so aufeinander abgestimmt, dass es über den gesamten Zeitraum der Nutzung des beschichteten Gerätes zu einer konstanten Silberfreisetzung kommt. Diese Verfahren sind durch die Verwendung mehrerer Silbersalze und mehrerer Polymere umständlich.

Weitere Verfahren unter Verwendung von Silberionen beschreiben die WO 2001/037670 A und US 2003/0147960 A. WO 2001/037670 A betrifft dabei eine antimikrobielle Formulierung, die Silberionen in Zeolithen komplexiert. US 2003/0147960 A beschreibt Beschichtungen, in denen Silberionen in einer Mischung aus hydrophilen und hydrophoben Polymeren gebunden sind.

Die beschriebenen Verfahren unter Verwendung von Silbersalzen weisen die zuvor erwähnten Nachteile auf und sind darüber hinaus von ihrer Durchführung her kompliziert und daher vom Aufwand der Herstellung teuer, so dass weiterhin ein Bedarf an verbesserten silberhaltigen Beschichtungen bezüglich des Herstellungsprozesses und der Wirksamkeit besteht.

Eine interessante Möglichkeit zur antimikrobiellen Ausrüstung von Kunststoffen ist die Verwendung von nanokristallinen Silberteilchen. Der Vorteil zum Beschichten mit metallischem Silber liegt in der im Verhältnis zum Volumen viel größeren Oberfläche des nanokristallinen Silbers, was zu einer erhöhten Freisetzung von Silberionen im Vergleich zu einer metallischen Silberbeschichtung führt.

Furno et al., Journal of Antimicrobial Chemotherapy 2004, 54, S. 1019-1024 beschreiben ein Verfahren, welches unter Verwendung von überkritischem Kohlendioxid nanokristallines Silber in Silikonoberflächen imprägniert. Dieses Verfahren ist aufgrund des komplizierten Imprägnierprozesses teuer und nicht leicht anwendbar.

Ferner sind verschiedene Verfahren bekannt, um nanokristallines Silber in Kunststoffe einzubauen. So beschreiben die WO 01/09229 A1, die WO 2004/024205 A1, die EP 0 711 113 A und Münstedt et al., Advanced Engineering Materials 2000, 2(6), Seiten 380 bis 386 den Einbau von nanokristallinem Silber in thermoplastische Polyurethane. Dabei werden Pellets aus einem handelsüblichen thermoplastischen Polyurethan in Lösung mit kolloidalem Silber durch Tränkung versetzt. Zur Erhöhung der antimikrobiellen Wirksamkeit erwähnen WO 2004/024205 A1 und DE 103 51 611 A1 zusätzlich, dass Bariumsulfat als Additiv verwendet werden kann. Aus den dotierten Polyurethanpellets werden dann durch Extrusion die entsprechenden Produkte wie Katheter hergestellt. Diese in den Veröffentlichungen beschriebene Vorgehensweise ist nachteilig, da die Menge an Silber, welche an den Polyurethanpellets nach dem Eintauchen verbleibt, nicht konstant ist bzw. nicht vorbestimmt werden kann. Der effektive Silbergehalt der resultierenden Produkte muss daher abschließend, d.h. nach Herstellung der Endprodukte, bestimmt werden. Demgegenüber ist ein Vorgehensweise, mit welcher die effektive Silbermenge, welche in dem resultierenden Endprodukt vorgesehen sein soll, genau eingestellt wird, aus diesen Veröffentlichungen nicht bekannt.

Ein ähnliches Verfahren beschreibt die EP 0 433 961 A. Auch hier wird eine Mischung aus einem thermoplastischen Polyurethan (Pellethan), Silberpulver und Bariumsulfat gemischt und extrudiert.

Ein Nachteil dieses Verfahrens ist der relativ große Bedarf an Silber, welches im gesamten Kunststoffkörper verteilt wird. Dieses Verfahren ist daher teuer, und durch den Einbau des kolloidalen Silbers in der ganzen Kunststoffmatrix ist die Freisetzung des Silbers für eine ausreichende Wirksamkeit in Einzelfällen zu langsam. Die Verbesserung der Silberfreisetzung durch Zugabe von Bariumsulfat bedeutet einen weiteren kostenintensiven Arbeitsschritt.

Eine Beschichtungslösung aus einem thermoplastischen Polyurethan mit nanokristallinem Silber in einem organischen Lösemittel zur Herstellung von Gefäßprothesen beschreibt die WO 2006/032497 A. Die Struktur des Polyurethans wird nicht weiter spezifiziert, aber aufgrund der Beanspruchung von thermoplastischen Kunststoffen ist von der Verwendung von harnstofffreien Polyurethanen auszugehen. Die antibakterielle Wirkung wurde über das Wachstum adhärierter *Staphylococcus epidermidis*-Zellen auf der Prüfkörperoberfläche im Vergleich zu einer Kontrolle bestimmt. Die detektierte antibakterielle Wirkung der silberhaltigen Beschichtungen ist allerdings als schwach zu bewerten, da nur eine Wachstumsverzögerung von maximal 33,2 h (ausgehend von einem bestimmten Schwellenwachstum) gegenüber der Kontrolloberfläche festgestellt wurde. Für längere Anwendungen als Implantat oder Katheter ist diese Beschichtungsformulierung somit nicht geeignet.

Die oben diskutierten Publikationen legen nahe, dass Silber ein sehr interessantes antimikrobielles Material ist, dass aber die veröffentlichten technischen Lösungsvorschläge zur Herstellung von antimikrobiellen Oberflächen für Implantate oder Katheter noch keine befriedigenden Lösungen darstellen.

Polyurethanharnstoffe in organischer Lösung sind sehr interessante Beschichtungsmaterialien, da mit ihnen eine nahezu unbegrenzte Vielfalt an Filmeigenschaften eingestellt werden können. Als Alternative können Polyurethanharnstoffe auch in wässriger Dispersion hergestellt werden. Obwohl solche rein wässrigen Systeme für bestimmte toxikologische Betrachtungen eine Alternative darstellen, zeigt die Erfahrung, dass auch Beschichtungen von Polyurethanharnstoffen aus organischer Lösung ohne Restlösemittelgehalte und damit ohne toxische Eigenschaften, welche gegebenenfalls auf Rückstände der organischen Lösemittel zurückzuführen sind, hergestellt werden können.

Allerdings sind die aus dem Stand der Technik bekannten Beschichtungen hinsichtlich der Glätte der Oberfläche, hinsichtlich der Festigkeit der ausgebildeten Beschichtungen und hinsichtlich der Freisetzungsverhaltens der antimikrobiellen Wirkstoffe noch nicht zufriedenstellend.

Die Aufgabe der vorliegenden Erfindung ist die somit Bereitstellung von Beschichtungen, die antimikrobiell ausgerüstet sind und die zuvor genannten Nachteile vorzugsweise nicht aufweisen.

Insbesondere sollen die Beschichtungen eine glatte Oberfläche aufweisen.

Ferner sollen die Beschichtungen vorzugsweise eine ausreichende Festigkeit aufweisen.

Schließlich sollen die Beschichtungen vorzugsweise ein zufriendenstellendes Verhalten bei der Freisetzung der antimikrobiellen Wirkstoffe aufweisen.

Diese Aufgabe wird durch eine Lösung eines Polyurethanharnstoffes gelöst, welches als antimikrobiellen Wirkstoff einen silberhaltigen Bestandteil aufweist.

Erfindungsgemäß wurde herausgefunden, dass durch die Verwendung von Polyurethanharnstoffen in organischen Lösungsmitteln, die mit einem silberhaltigen Bestandteil versetzt sind, Beschichtungen erzeugt werden können, welche hinsichtlich der Glätte der Oberfläche, hinsichtlich der Festigkeit der ausgebildeten Beschichtungen und hinsichtlich der Freisetzungsverhaltens der antimikrobiellen Wirkstoffe zufriedenstellend sind. Entsprechende erfindungsgemäße Versuche sowie entsprechende Vergleichsversuche, welche diese Erkenntnis stützen, sind weiter unten beschrieben.

Polyurethanharnstoffe im Sinne der vorliegenden Erfindung sind polymere Verbindungen, die
(a) mindestens zwei Urethangruppen enthaltende Wiederholungseinheiten der folgenden allgemeinen Struktur 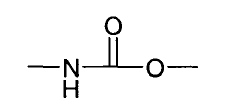 und
(b) mindestens eine Harnstoffgruppen enthaltende Wiederholungseinheit 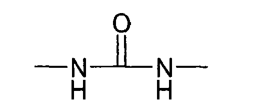 aufweisen.

Die erfindungsgemäßen Lösungen enthalten einen Polyurethanharnstoff, welcher im Wesentlichen keine ionische Modifizierung aufweisen. Hierunter wird im Rahmen der vorliegenden Erfindung verstanden, dass die erfindungsgemäß zu verwendenden Polyurethanharnstoffe im Wesentlichen keine ionischen Gruppen, wie insbesondere keine Sulfonat-, Carboxylat-, Phosphat- und Phosphonatgruppen, aufweisen.

Unter dem Begriff "im Wesentlichen keine ionische Gruppen" wird im Rahmen der vorliegenden Erfindung verstanden, dass die resultierenden Beschichtungen des Polyurethanharnstoffs ionische Gruppen mit einem Anteil von im Allgemeinen höchstens 2,50 Gew.-%, insbesondere höchstens 2,00 Gew.-%, vorzugsweise höchstens 1,50 Gew.-%, besonders bevorzugt höchstens 1,00 Gew.-%, speziell höchstens 0,50 Gew.-%, noch spezieller keine ionische Gruppen aufweist. Damit ist insbesondere bevorzugt, dass der Polyurethanharnstoff keine ionische Gruppen aufweist, da hohe Konzentrationen an Ionen in organischer Lösung dazu führen, dass das Polymer nicht mehr ausreichend löslich ist und somit keine stabilen Lösungen erhalten werden können. Falls das erfindungsgemäß verwendete Polyurethan ionische Gruppen aufweist, so handelt es sich bevorzugt um Carboxylate.

Die erfindungsgemäß zur Beschichtung der medizinischen Geräte vorgesehenen Polyurethanharnstoffe sind bevorzugt im Wesentlichen lineare Moleküle, können jedoch auch verzweigt sein, was jedoch weniger bevorzugt ist. Unter im Wesentlichen linearen Molekülen versteht man leicht anvernetzte Systeme, die eine Makropolyolkomponente als Aufbaukomponente, vorzugsweise ausgewählt aus der Gruppe, bestehend aus einem Polyetherpolyol, einem Polycarbonatpolyol und einem Polyesterpolyol, umfassen, die eine mittlere Funktionalität von vorzugsweise 1,7 bis 2,3, insbesondere 1,8 bis 2,2, besonders bevorzugt 1,9 bis 2,1, aufweisen.

Sollten Mischungen von Makropolyolen und gegebenenfalls Polyolen in dem Polyurethanharnstoff, wie nachstehend näher erläutert, verwendet werden, so bezieht sich die Funktionalität auf einen mittleren Wert, welcher sich durch die Gesamtheit der Makropolyole bzw. Polyole ergibt.

Das zahlenmittlere Molekulargewicht der erfindungsgemäß bevorzugt verwendeten Polyurethanharnstoffe beträgt vorzugsweise 1000 bis 200000, besonders bevorzugt von 5000 bis 100000. Dabei wird das zahlenmittlere Molekulargewicht gegen Polystyrol als Standard in Dimethylacetamid bei 30 °C gemessen.

### Polyurethanharnstoffe

Im Folgenden werden die erfindungsgemäß in organischer Lösung verwendeten Beschichtungssysteme auf Basis von Polyurethanharnstoffen näher beschrieben.
Die erfindungsgemäß verwendeten Polyurethanharnstoffe werden durch Umsetzung mindestens einer Makropolyolkomponente, mindestens einer Polyisoyanatkomponente, vorzugsweise mindestens einem Polyoxyalkylenether, mindestens einem Diamin und/oder einem Aminoalkohol und gegebenenfalls einer Polyolkomponente gebildet.

### (a) Makropolyolkomponente

Die Zusammensetzung des erfindungsgemäß vorgesehenen Polyurethanharnstoffes weist Einheiten auf, welche auf mindestens eine Makropolyolkomponente als Aufbaukomponente zurückgehen.

Die Makropolyolkomponente wird dabei im Allgemeinen ausgewählt aus der Gruppe, bestehend aus einem Polyetherpolyol, einem Polycarbonatpolyol, einem Polyesterpolyol und beliebigen Mischungen davon.

In einer bevorzugten Ausführungsform der vorliegenden Erfindung wird die Aufbaukomponente aus einem Polyetherpolyol oder einem Polycarbonatpolyol sowie aus Mischungen aus einem Polyetherpolyol und einem Polycarbonatpolyol gebildet.

In einer weiteren Ausführungsform der vorliegenden Erfindung wird die Aufbaukomponente eines Makropolyols aus einem Polyetherpolyol, insbesondere einem Polyetherdiol, gebildet. Polyetherpolyole und insbesondere Polyetherdiole sind hinsichtlich der Freisetzung von Silber besonders bevorzugt. Entsprechende erfindungsgemäße Versuche, welche diese Erkenntnisse stützen, sind weiter unten dargestellt.

Im Folgenden werden die einzelnen Makropolyolaufbaukomponenten näher beschrieben, wobei im Rahmen der vorliegenden Erfindung Polyurethanharnstoffe umfasst sind, welche sowohl nur eine Aufbaukomponente, ausgewählt aus im Allgemeinen Polyetherpolyolen, Polyesterpolyolen und Polycarbonatpolyolen, als auch Mischungen dieser Aufbaukomponenten umfassen. Ferner können die erfindungsgemäß vorgesehenen Polyurethanharnstoffe auch einen oder mehrere unterschiedliche Vertreter dieser Klassen an Aufbaukomponenten umfassen.

Die zuvor definierte Funktionalität der erfindungsgemäß vorgesehenen Polyurethanharnstoffe wird, wenn mehrere unterschiedliche Makropolyole und Polyole oder Polyamine (welche weiter unten unter c) und e) beschrieben sind), im dem Polyurethanharnstoff zugegen sind, als die mittlere Funktionalität verstanden.

### Polyetherpolyol

Die in Frage kommenden, Hydroxylgruppen aufweisenden Polyether sind solche, die durch Polymerisation von cyclischen Ethern wie Ethylenoxid, Propylenoxid, Butylenoxid, Tetrahydrofuran, Styroloxid oder Epichlorhydrin mit sich selbst, z. B. in Gegenwart von BF₃ oder basischen Katalysatoren, oder durch Anlagerung dieser Ringverbindungen, gegebenenfalls im Gemisch oder nacheinander, an Startkomponenten mit reaktionsfähigen Wasserstoffatomen wie Alkoholen und Aminen oder Aminoalkoholen, z. B. Wasser, Ethylenglykol, Propylenglykol-1,2 oder Propylenglykol-1,3, hergestellt werden.

Bevorzugte hydroxylgruppenhaltige Polyether sind solche auf Basis von Ethylenoxid, Propylenoxid oder Tetrahydrofuran oder Mischungen dieser cyclischen Ether. Ganz besonders bevorzugte hydroxylgruppenhaltige Polyether sind solche auf Basis von polymerisiertem Tetrahydrofuran. Es können noch andere hydroxylgruppenhaltige Polyether wie auf Basis Ethylenoxid oder Propylenoxid zugegeben werden, wobei dann aber die Polyether auf Basis von Tetrahydrofuran mindestens zu vorzugsweise 50 Gew.-% enthalten sind.

### Polycarbonatpolyol

Als Hydroxylgruppen aufweisende Polycarbonate kommen Polycarbonate des durch OH-Zahl bestimmten Molekulargewichts von vorzugsweise 400 bis 6000 g/mol, besonders bevorzugt 500 bis 5000 g/mol, insbesondere von 600 bis 3000 g/mol in Frage, die beispielsweise durch Reaktion von Kohlensäurederivaten, wie Diphenylcarbonat, Dimethylcarbonat oder Phosgen, mit Polyolen, vorzugsweise Diolen, erhältlich sind. Als derartige Diole kommen beispielsweise Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentan-1,3-diol, Di-, Tri- oder Tetraethylenglykol, Dipropylenglykol, Polypropylenglykole, Dibutylenglykol, Polybutylenglykole, Bisphenol A, Tetrabrombisphenol A aber auch Lacton-modifizierte Diole in Frage.

Bevorzugt enthält die Diolkomponente 40 bis 100 Gew.-% Hexandiol, bevorzugt 1,6-Hexandiol und/oder Hexandiol-Derivate, vorzugsweise solche, die neben endständigen OH-Gruppen Ether- oder Estergruppen aufweisen, z. B. Produkte, die durch Umsetzung von 1 Mol Hexandiol mit mindestens 1 Mol, bevorzugt 1 bis 2 Mol Caprolacton oder durch Veretherung von Hexandiol mit sich selbst zum Di- oder Trihexylenglykol erhalten wurden. Auch Polyether-Polycarbonatdiole können eingesetzt werden. Die Hydroxylpolycarbonate sollten im wesentlichen linear sein. Sie können jedoch gegebenenfalls durch den Einbau polyfunktioneller Komponenten, insbesondere niedermolekularer Polyole, leicht verzweigt werden. Hierzu eignen sich beispielsweise Glycerin, Trimethylolpropan, Hexantriol-1,2,6, Butantriol-1,2,4, Trimethylolpropan, Pentaerythrit, Chinit, Mannit, Sorbit, Methylglykosid oder 1,3,4,6-Dianhydrohexite. Bevorzugt sind solche Polycarbonate auf Basis von Hexandiol-1,6, sowie modifizierend wirkenden Co-Diolen wie z. B. Butandiol-1,4 oder auch von ∈-Caprolacton. Weitere bevorzugte Polycarbonatdiole sind solche auf Basis von Mischungen aus Hexandiol-1,6 und Butandiol-1,4.

Das Polycarbonat ist vorzugsweise im Wesentlichen linear ausgebildet und weist nur eine geringfügige dreidimensionale Vernetzung auf, so dass Polyurethanharnstoffe gebildet werden, welche die zuvor genannte Spezifikation aufweisen.

### Polyesterpolyol

Die in Frage kommenden Hydroxylgruppen aufweisenden Polyester sind beispielsweise Umsetzungsprodukte von mehrwertigen, vorzugsweise zweiwertigen Alkoholen mit mehrwertigen, vorzugsweise zweiwertigen Polycarbonsäuren. Anstelle der freien Carbonsäuren können auch die entsprechenden Polycarbonsäureanhydride oder entsprechende Polycarbonsäureester von niedrigen Alkoholen oder deren Gemische zur Herstellung der Polyester verwendet werden.

Die Polycarbonsäuren können aliphatischer, cycloaliphatischer, aromatischer und/oder heterocyclischer Natur sein und gegebenenfalls, z. B. durch Halogenatome, substituiert sein und/oder ungesättigt sein. Bevorzugt sind aliphatische und cycloaliphatische Dicarbonsäuren. Als Beispiele hierfür seien genannt:

Bernsteinsäure, Adipinsäure, Azelainsäure, Sebacinsäure, Phthalsäure, Tetrachlorphthalsäure, Isophtalsäure, Terephthalsäure, Tetrahydrophthalsäure, Hexahydrophthalsäure, Cyclohexandicarbonsäure, Itaconsäure, Sebacinsäure, Glutarsäure, Korksäure, 2-Methylbernsteinsäure, 3,3-Diethylglutarsäure, 2,2-Dimethylbernsteinsäure, Maleinsäure, Malonsäure, Fumarsäure oder Terephthalsäuredimethylester. Anhydride dieser Säuren sind ebenfalls brauchbar, soweit sie existieren. Beispiele hierfür sind Maleinsäureanhydrid, Phthalsäureanhydrid, Tetrahydrophthalsäureanhydrid, Glutarsäureanhydrid, Hexahydrophthalsäureanhydrid und Tetrachlorphthalsäureanhydrid.

Als gegebenenfalls in kleinen Mengen mitzuverwendende Polycarbonsäure sei hier Trimellitsäure genannt.

Als mehrwertige Alkohole kommen vorzugsweise Diole zur Anwendung. Beispiele solcher Diole sind z. B. Ethylenglykol, Propylenglykol-1,2, Propylenglykol-1,3, Butandiol-1,4, Butandiol-2,3, Diethylenglykol, Triethylenglykol, Hexandiol-1,6, Octandiol-1,8, Neopentylglykol, 2-Methyl-1,3-propandiol oder Hydroxypivalinsäureneopentylglykolester in Frage. Auch Polyesterdiole aus Lactonen, z. B. ∈-Caprolacton, sind einsetzbar. Als gegebenenfalls mit einzusetzende Polyole sind hier beispielsweise Trimethylolpropan, Glycerin, Erythrit, Pentaerythrit, Trimethylolbenzol oder Trishydroxyethylisocyanurat zu nennen.

### (b) Polyisocyanat

Die erfindungsgemäß vorgesehenen Polyurethanharnstoffe weisen Einheiten auf, welche auf mindestens ein Polyisocyanat als Aufbaukomponente zurückgehen.

Als Polyisocyanate (b) können alle dem Fachmann bekannten aromatischen, araliphatischen, aliphatischen und cycloaliphatischen Isocyanate einer mittleren NCO-Funktionalität >_ 1, bevorzugt ≥ 2 einzeln oder in beliebigen Mischungen untereinander eingesetzt werden, wobei es unerheblich ist, ob diese nach Phosgen- oder phosgen-freien Verfahren hergestellt wurden. Diese können auch Iminooxadiazindion-, Isocyanurat-, Uretdion-, Urethan-, Allophanat-, Biuret-, Harnstoff-, Oxadiazintrion, Oxazolidinon-, Acylharnstoff- und/oder Carbodiimid-Strukturen aufweisen. Die Polyisocyanate können einzeln oder in beliebigen Mischungen untereinander eingesetzt werden.

Bevorzugt werden Isocyanate aus der Reihe der aliphatischen oder cycloaliphatischen Vertreter eingesetzt, wobei diese ein Kohlenstoffgrundgerüst (ohne die enthaltenen NCO-Gruppen) von 3 bis 30, bevorzugt 4 bis 20 Kohlenstoffatomen aufweisen.

Besonders bevorzugte Verbindungen der Komponente (b) entsprechen der vorstehend genannten Art mit aliphatisch und/oder cycloaliphatisch gebundene NCO-Gruppen wie beispielsweise Bis-(isocyanatoalkyl)ether, Bis- und Tris-(isocyanatoalkyl)benzole, -toluole, sowie -xylole, Propandiisocyanate, Butandiisocyanate, Pentandiisocyanate, Hexandiisocyanate (z.B. Hexamethylendiisocyanat, HDI), Heptandiisocyanate, Octandiisocyanate, Nonandiisocyanate (z.B. Trimethyl-HDI (TMDI) in der Regel als Gemisch der 2,4,4- und 2,2,4-Isomeren), Nonantriisocyanate (z.B. 4-Isocyanatomethyl-1,8-octandiisocyanat), Dekandiisocyanate, Dekantriisocyanate, Undekandiisocyanate, Undekantriisocyanate, Dodecandiisocyanate, Dodekantriisocyanate, 1,3- sowie 1,4-Bis-(isocyanatomethyl)cyclohexane (H₆XDI), 3-Isocyanatomethyl-3,5,5-trimethylcyclohexylisocyanat (Isophorondiisocyanat, IPDI), Bis-(4-isocyanatocyclohexyl)methan (H₁₂MDI) oder Bis-(isocyanatomethyl)norbornan (NBDI).

Ganz besonders bevorzugte Verbindungen der Komponente (b) sind Hexamethylendiisocyanat (HDI), Trimethyl-HDI (TMDI), 2-Methylpentan-1,5-diisocyanat (MPDI), Isophorondüsocyanat (IPDI), 1,3- sowie 1,4-Bis(isocyanatomethyl)cyclohexan (H₆XDI), Bis(isocyanatomethyl)-norbornan (NBDI), 3(4)-Isocyanatomethyl-1-methyl-cyclohexylisocyanat (IMCI) und/oder 4,4'-Bis(isocyanatocyclohexyl)methan (H₁₂MDI) oder Gemische dieser Isocyanate. Weitere Beispiele sind Derivate aus den vorstehenden Diisocyanaten mit Uretdion-, Isocyanurat-, Urethan-, Allophanat-, Biuret-, Iminooxadiazindion- und/oder Oxadiazintrionstruktur mit mehr als zwei NCO-Gruppen.
Die Menge an Bestandteil (b) in dem erfindungsgemäß vorgesehenen Polyurethanharnstoff beträgt vorzugsweise 1,0 bis 4,0 mol, besonders bevorzugt 1,2 bis 3,8 mol, insbesondere 1,5 bis 3,5 mol, jeweils bezogen auf den Bestandteil (a) des Polyurethanharnstoffs.

### (c) Diamin oder Aminoalkohol

Die erfindungsgemäß vorgesehenen Polyurethanharnstoffe weisen Einheiten auf, welche auf mindestens ein Diamin oder ein Aminoalkohol als Aufbaukomponente zurückgehen und als sogenannte Kettenverlängerer (c) fungieren.

Solche Kettenverlängerer sind beispielsweise Di- oder Polyamine sowie Hydrazide, z.B. Hydrazin, Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 1,3- und 1,4-Xylylendiamin, α,α,α',α'-Tetramethyl-1,3- und -1,4-xylylendiamin und 4,4'-Diaminodicyclohexylmethan, Dimethylethylendiamin, Hydrazin, Adipinsäuredihydrazid, 1,4-Bis(aminomethyl)cyclohexan, 4,4'-Diamino-3,3'-dimethyldicyclohexylmethan und andere (C₁ - C₄)-Di- und Tetraalkyldicyclohexylmethan, z. B. 4,4'-Diamino-3,5-diethyl-3',5'-diisopropyldicyclohexylmethan.

Als Diamine oder Aminoalkohole kommen im Allgemeinen niedermolekulare Diamine oder Aminoalkohole in Betracht, die aktiven Wasserstoff mit gegenüber NCO-Gruppen unterschiedlicher Reaktivität enthalten, wie Verbindungen, die neben einer primären Aminogruppe auch sekundäre Aminogruppen oder neben einer Aminogruppe (primär oder sekundär) auch OH-Gruppen aufweisen. Beispiele hierfür sind primäre und sekundäre Amine, wie 3-Amino-1-Methylaminopropan, 3-Amino-1-ethylaminopropan, 3-Amino-1-cyclohexylaminopropan, 3-Amino-1- Methylaminobutan, weiterhin Aminoalkohole, wie N-Aminoethylethanolamin, Ethanolamin, 3-Aminopropanol, Neopentanolamin und besonders bevorzugt Diethanolamin.

Der Bestandteil (c) des erfindungsgemäß vorgesehenen Polyurethanharnstoffes kann bei dessen Herstellung als Kettenverlängerer eingesetzt werden.

Die Menge an Bestandteil (c) in dem erfindungsgemäß vorgesehenen Polyurethanharnstoff beträgt vorzugsweise 0,05 bis 3,0 mol, besonders bevorzugt 0,1 bis 2,0 mol, insbesondere 0,2 bis 1,5 mol, jeweils bezogen auf den Bestandteil (a) des Polyurethanharnstoffes.

### (d) Polyoxyalkylenether

Die erfindungsgemäß vorgesehenen Polyurethanharnstoffe weisen vorzugsweise Einheiten auf, welche auf einen Polyoxyalkylenether als Aufbaukomponente zurückgehen.

Bei dem Polyoxyalkylenether handelt es sich vorzugsweise um ein Copolymer aus Polyethylenoxid und Polypropylenoxid. Diese Copolymereinheiten liegen als Endgruppen in dem Polyurethanharnstoff vor und bewirken eine Hydrophilierung des Polyurethanharnstoffes.

Geeignete nichtionisch hydrophilierende Verbindungen entsprechend der Definition der Komponente (d) sind z. B. Polyoxyalkylenether, die mindestens eine Hydroxy- oder Aminogruppe enthalten. Diese Polyether enthalten im Allgemeinen einen Anteil von 30 Gew.-% bis 100 Gew.-% an Bausteinen, die vom Ethylenoxid abgeleitet sind.

Nichtionisch hydrophilierende Verbindungen (d) sind beispielsweise einwertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ullmanns Enzyklopädie der technischen Chemie, 4. Auflage, Band 19, Verlag Chemie, Weinheim S. 31-38).

Geeignete Startermoleküle sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylenglykolmonobutylether als Startermolekül verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Bei den Polyalkylenoxidpolyetheralkoholen handelt es sich entweder um reine Polyethylenoxidpolyether oder gemischte Polyalkylenoxidpolyether, deren Alkylenoxideinheiten zu mindestens 30 mol-%, bevorzugt zu mindestens 40 mol-% aus Ethylenoxideinheiten bestehen. Bevorzugte nichtionische Verbindungen sind monofunktionelle gemischte Polyalkylenoxidpolyether, die mindestens 40 mol-% Ethylenoxid- und maximal 60 mol-% Propylenoxideinheiten aufweisen.

Erfindungsgemäß konnte gezeigt werden, dass sich die Polyurethanharnstoffe mit Endgruppen, die auf gemischten Polyoxyalkylenether aus Polyethylenoxid und Polypropylenoxid basieren, insbesondere dazu eignen, Beschichtungen zu erzeugen, die den antimikrobiellen Wirkstoff besonders effizient freisetzen. Dieses wird, wie weiter unten dargestellt, auch experimentell gezeigt.

Wenn die Alkylenoxide Ethylenoxid und Propylenoxid eingesetzt werden, können sie in beliebiger Reihenfolge oder auch im Gemisch bei der Alkoxylierungsreaktion eingesetzt werden.

Das mittlere Molgewicht des Polyoxyalkylenethers beträgt vorzugsweise 500 g/mol bis 5000 g/mol, besonders bevorzugt 1000 g/mol bis 4000 g/mol, insbesondere 1000 bis 3000 g/mol.

Die Menge an Bestandteil (d) in dem erfindungsgemäß vorgesehenen Polyurethanharnstoff beträgt vorzugsweise 0,01 bis 0,5 mol, besonders bevorzugt 0,02 bis 0,4 mol, insbesondere 0,04 bis 0,3 mol, jeweils bezogen auf den Bestandteil (a) des Polyurethanharnstoffes.

### (e) Polyole

In einer weiteren Ausführungsform umfasst der erfindungsgemäß vorgesehene Polyurethanharnstoff zusätzlich Einheiten, welche auf mindestens ein Polyol als Aufbaukomponente zurückgehen. Bei diesen Polyolaufbaukomponenten handelt es sich im Vergleich zu dem Makropolyol um relativ kurzkettige Aufbaukomponenten, die eine Versteifung durch zusätzliche Hartsegmente hervorrufen können.

Die zum Aufbau der Polyurethanharnstoffe eingesetzten niedermolekularen Polyole (e) bewirken in der Regel daher eine Versteifung und/oder eine Verzweigung der Polymerkette. Das Molekulargewicht beträgt vorzugsweise 62 bis 500 g/mol, besonders bevorzugt 62 bis 400 g/mol, insbesondere 62 bis 200 g/mol.

Geeignete Polyole können aliphatische, alicyclische oder aromatische Gruppen enthalten. Genannt seien hier beispielsweise die niedermolekularen Polyole mit bis zu etwa 20 Kohlenstoffatomen je Molekül, wie z. B. Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), sowie Trimethylolpropan, Glycerin oder Pentaerythrit und Mischungen dieser und gegebenenfalls auch weiterer niedermolekularer Polyole. Auch Esterdiole wie z. B. α-Hydroxybutyl-ε-hydroxy-capronsäureester, ω-Hydroxyhexyl-γhydroxybuttersäureester, Adipinsäure-(β-hydroxyethyl)ester oder Terephthalsäure-bis(β-hydroxyethyl)-ester können verwendet werden.
Die Menge an Bestandteil (e) in dem erfindungsgemäß vorgesehenen Polyurethanharnstoff beträgt vorzugsweise 0,1 bis 1,0 mol, besonders bevorzugt 0,2 bis 0,9 mol, insbesondere 0,2 bis 0,8 mol, jeweils bezogen auf den Bestandteil (a) des Polyurethanharnstoffes.

### (f) Weitere amin- und/oder hydroxventhaltende Bausteine (Aufbaukomponente)

Die Umsetzung der isocyanathaltigen Komponente (b) mit den hydroxy- oder aminfunktionellen Verbindungen (a), (c), (d) und gegebenenfalls (e) erfolgt üblicherweise unter Einhaltung eines leichten NCO-Überschusses gegenüber den reaktiven Hydroxy- oder Aminverbindungen. In diesem Fall verbleiben am Endpunkt der Reaktion durch Erreichen einer Zielviskosität immer noch Reste an aktivem Isocyanat. Diese Reste müssen blockiert werden, damit nicht eine Reaktion mit großen Polymerketten stattfindet. Eine solche Reaktion führt zur dreidimensionalen Vernetzung und Vergelung des Ansatzes. Die Verarbeitung einer solchen Beschichtungslösung ist nur eingeschränkt oder nicht mehr möglich. Üblicherweise enthalten die Ansätze hohe Mengen an Alkoholen. Diese Alkohole blockieren innerhalb von mehreren Stunden beim Stehen lassen oder beim Rühren des Ansatzes bei Raumtemperatur die noch verbliebenen Isocyanatgruppen.

Will man aber den noch verbleibenden Restisocyanatgehalt schnell blockieren, können die erfindungsgemäß vorgesehenen Polyurethanharnstoffe auch Monomere (f) enthalten, die sich jeweils an den Kettenenden befinden und diese abschließen.

Diese Bausteine leiten sich zum einen von monofunktionellen, mit NCO-Gruppen reaktiven Verbindungen ab, wie Monoaminen, insbesondere mono-sekundären Aminen oder Monoalkoholen. Genannt seien hier beispielsweise Ethanol, n-Butanol, Ethylenglykolmonobutylether, 2-Ethylhexanol, 1-Octanol, 1-Dodecanol, 1-Hexadecanol, Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin und geeignete substituierte Derivate davon.

Da die Bausteine (f) im Wesentlichen in den erfindungsgemäßen Beschichtungen dazu verwendet werden, den NCO-Überschuss zu vernichten, hängt die erforderliche Menge im Wesentlichen von der Menge des NCO-Überschusses ab und kann nicht allgemein spezifiziert werden.

Bevorzugt wird während der Synthese auf diese Bausteine verzichtet. Noch nicht umgesetztes Isocyanat wird dabei vorzugsweise durch die in sehr großen Konzentrationen enthaltenen Lösungsmittelalkohole zu terminalen Urethanen umgesetzt.

### (g) Weitere Bestandteile

Obwohl die erfindungsgemäßen Lösungen der Polyurethanharnstoffe aufgrund der antimikrobiellen Ausrüstung durch den silberhaltigen Bestandteil bereits ausreichend funktionalisiert sind, kann es im Einzelfall von Vorteil sein, weitere Funktionalisierungen in die Lösung zu integrieren. Diese weiteren möglichen Funktionalisierungen werden nun im Folgenden näher beschrieben.

Die erfindungsgemäß vorgesehenen Polyurethanharnstoff-Lösungen können darüber hinaus weitere, für den angestrebten Zweck übliche Bestandteile wie Additive und Füllstoffe, enthalten. Ein Beispiel hierfür sind pharmakologische Wirkstoffe, Arzneimittel und Additive, welche die Freisetzung von pharmakologischen Wirkstoffen fördern ("drug-eluting-Additive").

Pharmakologische Wirkstoffe bzw. Arzneimittel, welche in den erfindungsgemäßen Beschichtungen auf den medizinischen Geräten verwendet werden können, sind im Allgemeinen beispielsweise thromboresistente Mittel, antibiotische Mittel, Antitumormittel, Wachtumshormone, Antivirusmittel, antiangiogene Mittel, angiogene Mittel, antimitotische Mittel, entzündungshemmende Mittel, Zellzyklus-regulierende Mittel, genetische Mittel, Hormone, sowie deren Homologe, Derivate, Fragmente, pharmazeutische Salze und Kombinationen davon.

Spezifische Beispiele solcher pharmakologischer Wirkstoffe bzw. Arzneimittel schließen somit thromboresistente (nichtthrombogene) Mittel bzw. andere Mittel zur Unterdrückung einer akuten Thrombose, Stenose oder späten Re-Stenose der Arterien ein, beispielsweise Heparin, Streptokinase, Urokinase, Gewebe-Plasminogen-Aktivator, Anti-Thromboxan-B₂-Mittel; Anti-B-Thromoboglobulin, Prostaglandin-E, Aspirin, Dipyridimol, Anti-Thromboxan-A₂-Mittel, muriner monoklonaler Antikörper 7E3, Triazolopyrimidin, Ciprosten, Hirudin, Ticlopidin, Nicorandil usw. Ein Wachstumsfaktor kann als ebenfalls als ein Arzneimittel benutzt werden, um unterintimale fibromuskülare Hyperplasie an der arteriellen Stenosestelle zu unterdrücken, bzw. es kann jeder beliebige andere Hemmstoff des Zellwachstums an der Stenosestelle benutzt werden.

Der pharmakologische Wirkstoff bzw. das Arzneimittel kann auch aus einem Vasodilatator bestehen, um Vasospasmus entgegen zu wirken, zum Beispiel ein Antispasmusmittel wie Papaverin. Das Arzneimittel kann ein vasoaktives Mittel an sich sein, wie Calciumantagonisten, oder α- und β-adrenergische Agonisten oder Antagonisten. Zusätzlich kann das therapeutische Mittel ein biologisches Haftmittel wie Cyanoacrylat in medizinischer Qualität oder Fibrin, welche beispielsweise für das Ankleben einer Gewebeklappe an die Wand einer Koronararterie verwendet wird, sein.

Das therapeutische Mittel kann ferner ein antineoplastisches Mittel wie 5-Fluorouracil, vorzugsweise mit einem kontrollierenden freisetzenden Träger für das Mittel, sein (z.B. für die Anwendung eines fortdauernden kontrollierten freisetzenden antineoplastischen Mittels an einer Tumorstelle).

Das therapeutische Mittel kann ein Antibiotikum, vorzugsweise in Kombination mit einem kontrollierenden freisetzenden Träger für die fortdauernde Freisetzung aus der Beschichtung eines medizinischen Geräts an einen lokalisierten Infektionsherd innerhalb des Körpers, sein. Ähnlich kann das therapeutische Mittel Steroide für den Zweck des Unterdrückens einer Entzündung in lokalisiertem Gewebe oder aus anderen Gründen enthalten.

Spezifische Beispiele geeigneter Arzneimittel umfassen:
(a) Heparin, Heparinsulfat, Hirudin, Hyaluronsäure, Chondroitinsulfat, Dermatansulfat, Keratansulfat, lytische Mittel, einschließlich Urokinase und Streptokinase, deren Homologe, Analoge, Fragmente, Derivate und pharmazeutische Salze davon;
(b) antibiotische Mitteln wie Penicilline, Cephalosprine, Vacomycine, Aminoglycoside, Quinolone, Polymxine, Erythromycine; Tertracycline, Chloramphenicole, Clindamycine, Lincomycine, Sulfonamide, deren Homologe, Analoge, Derivate, pharmazeutische Salze und Mischungen davon;
(c) Paclitaxel, Docetaxel, Immunsuppressiva wie Sirolimus oder Everolimus, Alkylierungsmittel einschließlich Mechlorethamin, Chlorambucil, Cyclophosphamid, Melphalan und Ifosfamid; Antimetaboliten einschließlich Methotrexat, 6-Mercaptopurin, 5-Fluorouracil und Cytarabin; Pflanzenalkoide einschließlich Vinblastin; Vincristin und Etoposid; Antibiotika einschließlich Doxorubicin, Daunomycin, Bleomycin und Mitomycin; Nitrosurea einschließlich Carmustin und Lomustin; anorganische Ionen einschließlich Cisplatin; biologische Reaktionsmodifikatoren einschließlich Interferon; angiostatinische Mittel und endostatinische Mittel; Enzyme einschließlich Asparaginase; und Hormone einschließlich Tamoxifen und Flutamid, deren Homologe, Analoge, Fragmente, Derivate, pharmazeutische Salze und Mischungen davon; und
(d) antivrale Mittel wie Amantadin, Rimantadin, Rabavirin, Idoxuridin, Vidarabin, Trifluridin, Acyclovir, Ganciclocir, Zidovudin, Phosphonoformate, Interferone, deren Homologe, Analoge, Fragmente, Derivate, pharmazeutische Salze und Mischungen davon; und
(e) entzündungshemmende Mittel wie beispielsweise Ibuprofen, Dexamethason oder Methylprednisolon.

Weitere übliche Zusatzstoffe und Hilfsmittel wie Verdickungsmittel, Griffhilfsmittel, Pigmente, Farbstoffe, Mattierungsmittel, UV-Stabilisatoren, phenolische Antioxidantien, Lichstabilisatoren, Hydrophobierungsmittel und/oder Verlaufshilfsmittel können ebenfalls in der erfindungsgemäß vorgesehenen Beschichtung mitverwendet werden.

### (h) Antimikrobiell wirkendes Silber

Die erfindungsgemäße Polyurethanharnstoff-Lösung umfasst einen antimikrobiellen silberhaltigen Bestandteil.

Unter einem antimikrobiellen silberhaltigen Bestandteil wird ein Bestandteil der erfindungsgemäßen Polyurethan-Lösung verstanden, welcher einen wirksamen Gehalt an einer Komponente aufweist, die Silber und/oder Silberionen unter bestimmten Bedingungen freisetzt und somit antimikrobiell wirkt. Bevorzugte silberhaltige Bestandteile im Sinne der vorliegenden Erfindung werden nun beschrieben, wobei die vorliegende Erfindung nicht auf diesen speziellen Systeme beschränkt ist.

Die biozide Wirkung von Silber beruht auf der Wechselwirkung von Silberionen mit Bakterien. Um eine möglichst große Anzahl von Silberionen aus elementarem Silber erzeugen zu können, ist eine große Oberfläche des Silbers vorteilhaft. Daher werden hauptsächlich für antimikoribielle Anwendungen hochporöse Silberpulver, Silber auf Trägermaterialien oder kolloidale Silber-Sole verwendet werden.

Zur Zeit kommerziell erhältlich sind beispielsweise Ag-lon (Silber in einem Zeolith, Agion, Wakefield, MA, USA), lonpure^{®} (Ag⁺ in Glas, Ciba Spezialitätenchemie GmbH, Lampertheim, Deutschland), Alphasan^{®} (AgZr-Phosphat, Milliken Chemical, Gent, Belgien), Irgaguard^{®} (Ag in Zeolith/Glas), Hygate^{®} (Silberpulver, Bio-Gate, Nürnberg, Deutschland), NanoSilver BG (Silber in Suspension) und Nanocid^{®} (Silber auf TiO₂, Pars Nano Nasb Co., Teheran, Iran).

Die Silberpulver werden vorzugsweise aus einer Gasphase erhalten, wobei eine Silberschmelze in Helium verdampft wird. Die daraus resultierenden Nanopartikel agglomerieren sofort und werden als hochporöse, gut filtrierbare Pulver erhalten. Der Nachteil diese Pulver besteht allerdings darin, dass sich die Agglomerate nicht mehr in Einzelpartikel dispergieren lassen.

Kolloidale Silber-Dispersionen werden durch Reduktion von Silbersalzen in organischen oder wässrigen Medium erhalten. Die Herstellung ist aufwendiger als die der Silberpulver, bietet aber den Vorteil, dass nicht agglomerierte Nanopartikel erhalten werden. Durch das Einarbeiten von nicht agglomerierten Nanopartikein in Beschichtungsmaterialien können transparente Filme erzeugt werden.

Für die erfindungsgemäßen, silberhaltigen Polyurethanharnstoff-Lösungen können beliebige Silberpulver oder kolloidale Silberdispersionen verwendet werden. Eine Vielzahl solcher Silbermaterialien ist kommerziell erhältlich.

Die zur Formulierung der erfindungsgemäßen, silberhaltigen Polyurethanharnstoff-Lösungen bevorzugt verwendeten Silbersole werden aus Ag₂O durch Reduktion mit einem Reduktionsmittel wie wässriger Formaldehydlösung nach vorhergehender Zugabe eines Dispergierhilfsmittels hergestellt. Dazu werden die Ag₂O-Sole zum Beispiel durch schnelles Vermischen von Silbernitratlösung mit NaOH durch schnelles Rühren batchweise oder durch Verwendung eines Mikromischers entsprechend der DE 10 2006 017 696 in einem kontinuierlichen Prozess hergestellt. Anschließend werden die Ag₂O-Nanopartikel mit Formaldehyd im Überschuss in einem Batch-Verfahren reduziert und abschließend durch Zentrifugation oder Membranfiltration, bevorzugt durch Membranfiltration, gereinigt. Besonderes vorteilhaft ist diese Produktionsweise, weil die Menge an auf der Oberfläche der Nanopartikel gebundenen organischen Hilfsmitteln hierbei gering gehalten werden kann. Man erhält eine Silbersoldispersion in Wasser mit einer mittleren Teilchengröße von ungefähr 10 bis 150 nm, besonders bevorzugt 20 bis 100 nm.

Für die Herstellung von antimikrobiell ausgerüsteten Beschichtungen können nanokristalline Silberteilchen mit einer mittleren Größe von 1 bis 1000 nm, bevorzugt 5 bis 500 nm, ganz besonders bevorzugt von 10 bis 250 nm eingesetzt werden. Diese Teilchengröße wird mittels Laserkorrelationsspektroskopie bestimmt.

Der Kristallinitätsgrad der eingesetzten Silberpartikel beträgt bevorzugt 50, besonders bevorzugt 70, ganz besonders bevorzugt 90 %.

Die Nanosilberteilchen sind in organischen Lösungsmitteln dispergiert. Für die Zugabe in organische Lösungen von Polyurethanharnstoffen muss das Wasser der nanokristallinen Silberdispersion durch ein organisches Lösungsmittel ausgetauscht werden. Vorzugsweise wird die Silberdispersion in den Lösungsmitteln aufgenommen, die auch zur Lösung der Polyurethanharnstoffe zur Anwendung kommen. Beispiele solcher Lösungsmittel sind aromatische Lösungsmittel wie Toluol, Alkohole wie Ethanol oder Isopropanol, organische Ester wie Essigsäureethylester oder Butylacetat sowie Ketone wie Aceton oder Methylethylketon. Auch Mischungen aus den genannten Lösungsmitteln sind zur Aufnahme der Silberdispersion geeignet. Die Herstellung der Beschichtungsrohmaterialien erfolgt durch Zugabe der Silberdispersion zu der Polyurethanlösung und anschließendes Homogenisieren durch Rühren oder Schütteln.
Die Menge an nanokristallinem Silber, bezogen auf die Menge an festem Polymer und gerechnet als Ag und Ag⁺, kann variabel eingestellt werden. Übliche Konzentrationen gehen von 0,1 bis 10 Gew.-%, bevorzugt von 0,3 bis 5 Gew.-%, besonders bevorzugt von 0,5 bis 3 Gew.-%.

Der Vorteil der erfindungsgemäßen silberhaltigen Polyurethane liegt im Vergleich zu vielen alternativen Verfahren in der sehr leichten Kombinationsfähigkeit der Polyurethanlösungen und der kolloidalen Silberdispersionen. Unterschiedliche Silberkonzentrationen können nach Bedarf für verschiedene Anwendungen leicht und exakt eingestellt werden. Viele Verfahren des Standes der Technik sind wesentlich aufwändiger und auch in der Dosierung der Silbermenge nicht so exakt wie das erfindungsgemäße Verfahren.

In einer besonders bevorzugten Ausführungsform liegt das antimikrobiell wirksames Silber in Form hochporöser Silberpulver, Silber auf Trägermaterialien oder in Form kolloidaler Silber-sole vor, wobei bezogen auf das feste Polyurethanpolymer 0,1 bis 10 Gew.-% an Silber enthalten ist.

In einer weiteren besonders bevorzugten Ausführungsform liegt das antimikrobiell wirksame Silber in Form kolloidaler Silbersole in wässrigem Medium oder in wassermischbaren organischen Lösungsmitteln mit einer Partikelgröße von 1 bis 1000 nm vor, wobei bezogen auf das feste Polyurethanpolymer 0,3 bis 5 Gew.-% zugegeben wird

In einer weiteren besonders bevorzugten Ausführungsform liegt das antimikrobiell wirksame Silber in Form kolloidaler Silbersole in wässrigem Medium mit einer mittleren Partikelgröße von 1 bis 500 nm, wobei bezogen auf das feste Polyurethanpolymer 0,5 bis 3 Gew.-% zugegeben wird.

### Polyurethanharnstoff-Zusammensetzung

In einer bevorzugten Ausführungsform umfasst die erfindungsgemäße Polyurethanharnstoff-Lösung einen Polyurethanharnstoff, welcher zumindest aufgebaut wird aus
a) mindestens einem Makropolyol;
b) mindestens einem Polyisocyanat;
c) mindestens einem Diamin oder einem Aminoalkohol; und
d) vorzugsweise mindestens einem monofunktionellen Polyoxyalkylenether; sowie
h) mindestens einen antimikrobiell wirksamen silberhaltigen Bestandteil.

In einer weiteren Ausführungsform der vorliegenden Erfindung umfasst die erfindungsgemäße Polyurethanharnstoff-Lösung einen Polyurethanharnstoff, welcher zumindest aufgebaut wird aus
a) mindestens einem Makropolyol;
b) mindestens einem Polyisocyanat;
c) mindestens einem Diamin oder einem Aminoalkohol;
d) mindestens einem monofunktionellen Polyoxyalkylenether; und
e) mindestens einem weiteren Polyol; sowie
h) mindestens einen antimikrobiell wirksamen silberhaltigen Bestandteil.

In einer weiteren Ausführungsform der vorliegenden Erfindung umfasst die erfindungsgemäße Polyurethanharnstoff-Lösung einen Polyurethanharnstoff, welcher zumindest aufgebaut wird aus
a) mindestens einem Makropolyol;
b) mindestens einem Polyisocyanat;
c) mindestens einem Diamin oder einem Aminoalkohol;
d) mindestens einem monofunktionellen Polyoxyalkylenether;
e) mindestens einem Polyol; und
f) mindestens einem amin- oder hydroxylhaltiges Monomer, welches sich an den Polymerkettenenden befindet;
   sowie
h) mindestens einen antimikrobiell wirksamen silberhaltigen Bestandteil.

Erfindungsgemäß besonders bevorzugt sind Lösungen von Polyurethanharnstoffen, die einen Polyurethanharnstoff enthalten, der aufgebaut wird aus
a) mindestens einem Makropolyol mit einem mittleren Molgewicht zwischen 400 g/mol und 6000 g/mol und einer Hydroxylfunktionalität von 1,7 bis 2,3 oder Mischungen solcher Makropolyole;
b) mindestens einem aliphatischen, cycloaliphatischen oder aromatischen Polyisocyanat oder Mischungen solcher Polyisocyanate in einer Menge pro Mol des Makropolyols von 1,0 bis 4,0 mol;
c) mindestens einem aliphatischen oder cycloaliphatischen Diamin oder mindestens ein Aminoalkohol als so genannte Kettenverlängerer oder Mischungen solcher Verbindungen in einer Menge pro Mol des Makropolyols von 0,05 bis 3,0 mol;
d) mindestens einem monofunktionellen Polyoxyalkylenether oder einer Mischung solcher Polyether mit einem mittleren Molgewicht zwischen 500 g/mol und 5000 g/mol in einer Menge pro Mol des Makropolyols von 0,01 bis 0,5 mol;
e) gegebenenfalls einem oder mehreren kurzkettigen aliphatischen Polyolen mit einem Molgewicht zwischen 62 g/mol und 500 g/mol in einer Menge pro Mol des Makropolyols von 0,1 bis 1 mol; und
f) gegebenenfalls amin- oder OH haltige Bausteine, die sich an den Polymerkettenenden befinden und diese abschließen; sowie
h) mindestens einen antimikrobiell wirksamen silberhaltigen Bestandteil enthalten.

Erfindungsgemäß weiter bevorzugt sind Polyurethanharnstoff-Lösungen, die einen Polyurethanharnstoff enthalten, der aufgebaut wird aus
a) mindestens einem Makropolyol mit einem mittleren Molgewicht zwischen 500 g/mol und 5000 g/mol und einer Hydroxylfunktionalität von 1,8 bis 2,2 oder Mischungen solcher Makropolyole;
b) mindestens einem aliphatischen, cycloaliphatischen oder aromatischen Polyisocyanat oder Mischungen solcher Polyisocyanate in einer Menge pro Mol des Makropolyols von 1,2 bis 3,8 mol;
c) mindestens einem aliphatischen oder cycloaliphatischen Diamin oder mindestens ein Aminoalkohol als so genannte Kettenverlängerer oder Mischungen solcher Verbindungen in einer Menge pro Mol des Makropolyols von 0,1 bis 2,0 mol;
d) mindestens einem monofunktionellen Polyoxyalkylenether oder einer Mischung solcher Polyether mit einem mittleren Molgewicht zwischen 1000 g/mol und 4000 g/mol in einer Menge pro Mol des Makropolyols von 0,02 bis 0,4 mol;
e) gegebenenfalls einem oder mehreren kurzkettigen aliphatischen Polyolen mit einem-Molgewicht zwischen 62 g/mol und 400 g/mol in einer Menge pro Mol des Makropolyols von 0,2 bis 0,9 mol; und
f) gegebenenfalls amin- oder OH haltige Bausteine, die sich an den Polymerkettenenden befinden und diese abschließen; sowie
h) mindestens einen antimikrobiell wirksamen silberhaltigen Bestandteil enthalten.

Erfindungsgemäß noch weiter bevorzugt sind Polyurethanharnstoff-Lösungen, die einen Polyurethanharnstoff enthalten, der aufgebaut wird aus
a) mindestens einem Makropolyol mit einem mittleren Molgewicht zwischen 600 g/mol und 3000 g/mol und einer Hydroxylfunktionalität von 1,9 bis 2,1 oder Mischungen solcher Makropolyole;
b) mindestens einem aliphatischen, cycloaliphatischen oder aromatischen Polyisocyanat oder Mischungen solcher Polyisocyanate in einer Menge pro Mol des Makropolyols von 1,5 bis 3,5 mol;
c) mindestens einem aliphatischen oder cycloaliphatischen Diamin oder mindestens ein Aminoalkohol als so genannte Kettenverlängerer oder Mischungen solcher Verbindungen in einer Menge pro Mol des Makropolyols von 0,2 bis 1,5 mol;
d) mindestens einem monofunktionellen Polyoxyalkylenether oder einer Mischung solcher Polyether mit einem mittleren Molgewicht zwischen 1000 g/mol und 3000 g/mol in einer Menge pro Mol des Makropolyols von 0,04 bis 0,3 mol, wobei eine Mischung aus Polyethylenoxid und Polypropylenoxid insbesondere bevorzugt ist; und
e) gegebenenfalls einem oder mehreren kurzkettigen aliphatischen Polyolen mit einem Molgewicht zwischen 62 g/mol und 400 g/mol in einer Menge pro Mol des Makropolyols von 0,2 bis 0,8 mol;
h) mindestens einen antimikrobiell wirksamen silberhaltigen Bestandteil enthalten. Die zuvor definierten Zusammensetzungen der Polyurethan-Lösunegn umfassen zusätzlich noch mindestens ein organisches Lösemittel.

Die organischen Lösemitteln können dabei beispielsweise ausgewählt werden aus der Gruppe, bestehend aus Dimethylformamid, N-Methylpyrrolidon, Dimethylacetamid, Tetramethylharnstoff, chlorierten Lösungsmitteln, aromatischen Lösemitteln, Ether, Ester, Ketone und Alkohole. Hierbei sind insbesondere aromatische Lösemittel, Ether, Ester, Ketone und Alkohole bevorzugt, wobei noch weiter bevorzugt Mischungen aus Toluol und Alkoholen sind.

Die erfindungsgemäßen Polyurethanharnstoff-Lösungen können zur Herstellung von Beschichtungen verwendet werden.

Beschichtet werden können dabei vielerlei Substrate wie Metalle, Textilien, Keramiken und Kunststoffe. Bevorzugt ist die Beschichtung von medizinischen Geräten, die aus Metallen oder Kunststoff gefertigt sind. Als Metalle sind beispielsweise zu nennen: Medizinischer Edelstahl und Nickel-Titan-Legierungen. Es sind viele Polymermaterialien denkbar, aus denen das medizinische Geräte aufgebaut sein kann, beispielsweise Polyamid; Polystyrol; Polycarbonat; Polyether; Polyester; Polyvinylacetat; natürliche und synthetische Kautschuke; Blockcopolymere aus Styrol und ungesättigten Verbindungen wie Ethylen, Butylen und Isopren; Polyethylen oder Copolymeren aus Polyethylen und Polypropylen; Silikon; Polyvinylchlorid (PVC) und Polyurethanen. Zur besseren Haftung des hydrophilen Polyurethane auf dem medizinischen Gerät können als Untergrund vor dem Auftragen dieser hydrophilen Beschichtungsmaterialien noch weitere geeignete Beschichtungen aufgetragen werden.

Insbesondere dienen die erfindungsgemäßen Polyurethan-Lösungen somit zur Beschichtung von medizinischen Geräten.

Die Bezeichnung "medizinisches Gerät" ist im Rahmen der vorliegenden Erfindung breit zu verstehen. Geeignete, nicht einschränkende Beispiele für medizinische Geräte (einschließlich Instrumente) sind Kontaktlinsen; Kanülen; Kathetern, zum Beispiel urologische Kathetern wie Blasenkathetern oder Harnleiterkathetern; zentralvenöse Katheter; venöse Kathetern oder Einlass- bzw. Auslass-Kathetern; Dilatationsballons; Kathetern für die Angioplastie und die Biopsie; Kathetern, die für das Einbringen eines Stents, eines Propf- oder eines Kavafilters verwendet werden; Ballonkathetern oder andere dehnbare medizinische Geräte; Endoskope; Larnygoskope; Trachealgeräte wie Endotrachealschläuche, Atemgeräte und andere Trachealabsauggeräte; bronchoalveolare Spülungskathetern; Kathetern, die bei der Koronarangioplastie verwendet werden; Führungsstäbe, Einführer und Ähnliches; Gefäßpfropfen; Schrittmacherteile; Cochleaimplantate; Zahnimplantatschläuche für die Nahrungszufuhr, Dränageschläuche; und Führungsdrähte.

Darüber hinaus können die erfindungsgemäßen Beschichtungslösungen zur Herstellung von Schutzbeschichtungen, zum Beispiel für Handschuhe, Stents und andere Implantate; extrakorporale (außerkörperliche) Blutschläuche (Blutführungsrohre); Membrane, zum Beispiel für die Dialyse; Blutfilter; Geräte für die Kreislaufunterstützung; Verbandsmaterial für die Wundpflege; Harnbeutel und Stomabeutel verwendet werden. Eingeschlossen sind auch Implantate, die ein medizinisch wirksames Mittel enthalten, wie medizinisch wirksame Mitteln für Stents oder für Ballonoberflächen oder für Kontrazeptiva.

Üblicherweise wird das medizinisches Gerät aus Kathetern, Endoskopen, Laryngoskopen, Endotrachealschläuchen, Ernährungsschläuchen, Führungsdrähten, Stents, und andere Implantate gebildet.

### Herstellung der Beschichtungen

Im Rahmen der vorliegenden Erfindung ist es vorgesehen, dass die Beschichtungen ausgehend von den oben beschriebenen Lösungen hergestellt werden.

Erfindungsgemäß hat sich herausgestellt, dass sich die Beschichtungen unterscheiden, je nachdem, ob die oben beschriebene Beschichtungszusammensetzung ausgehend von einer Dispersion oder einer Lösung hergestellt wird.

Dabei weisen die Beschichtungen dann Vorteile hinsichtlich mechanischer Stabilität auf, wenn sie ausgehend von Lösungen der oben beschriebenen Beschichtungszusammensetzungen erhalten werden. Außerdem sind die Filme aus organischen Lösungen wesentlich glatter als solche Filme, die aus wässrigen Polyurethandispersionen erhalten werden.

Die Beschichtungen können dabei mittels verschiedener Verfahren hergestellt werden. Geeignete Beschichtungstechniken sind hierfür beispielsweise Rakeln, Drucken, Transferbeschichten, Sprühen, Spincoating oder Tauchen.

Die organischen Polyurethanlösungen selbst können nach beliebigen Verfahren hergestellt werden.

Als bevorzugt hat sich jedoch folgende Verfahrensweise herausgestellt:

Zur Herstellung der erfindungsgemäß zur Beschichtung zu verwendenden Polyurethanharnstoff-Lösungen werden vorzugsweise das Makropolyol, das Polyisocyanat, gegebenenfalls der monofunktionelle Polyetheralkohol und gegebenenfalls das Polyol in der Schmelze oder in Lösung miteinander umgesetzt, bis alle Hydroxylgruppen verbraucht sind. Die dabei verwendete Stöchiometrie zwischen den einzelnen an der Umsetzung beteiligten Komponenten ergibt sich aus den zuvor erwähnten Mengenverhältnissen für die erfindungsgemäße Beschichtung.

Die Umsetzung erfolgt bei einer Temperatur von vorzugsweise zwischen 60 und 110 °C, besonders bevorzugt 75 bis 110 °C, insbesondere 90 bis 110 °C, wobei Temperaturen um 110 °C aufgrund der Geschwindigkeit der Umsetzung bevorzugt sind. Höhere Temperaturen können ebenfalls angewendet werden, allerdings besteht dann im Einzelfall und in Abhängigkeit der einzelnen verwendeten Bestandteile das Risiko, dass Zersetzungsprozesse und Verfärbungen in dem entstehenden Polymer auftreten.

Bei dem Prepolymer aus Isocyanat und allen Hydroxylgruppen aufweisenden Komponenten ist die Umsetzung in Schmelze bevorzugt, allerdings besteht die Gefahr, dass es zu hohen Viskositäten der ausreagierten Gemische kommt. In diesen Fällen empfiehlt es sich auch, Lösemittel hinzu zugegeben. Es sollte aber möglichst nicht mehr als ungefähr 50 Gew.-%

Lösemittel enthalten sein, da andernfalls die Verdünnung die Reaktionsgeschwindigkeit deutlich verlangsamt.

Bei der Umsetzung von Isocyanat und den Hydroxylgruppen aufweisenden Komponenten kann die Reaktion in der Schmelze in einem Zeitraum von 1 Stunde bis 24 Stunden erfolgen. Geringe Zugabe von Lösungsmittelmengen führen zu einer Verlangsamung, wobei die Umsetzungszeiträume jedoch in den gleichen Zeiträumen liegen.

Die Reihenfolge der Zugabe bzw. Umsetzung der einzelnen Bestandteile kann von der zuvor angegebenen Reihenfolge abweichen. Dieses kann insbesondere dann von Vorteil sein, wenn die mechanischen Eigenschaften der resultierenden Beschichtungen verändert werden sollen. Wenn man beispielsweise alle Hydroxylgruppen aufweisenden Komponenten gleichzeitig umsetzt, entsteht ein Gemisch aus Hart- und Weichsegmenten. Wenn man beispielsweise das niedermolekulare Polyol nach der Makropolyolkomponente zugibt, erhält man definierte Blöcke, was andere Eigenschaften der resultierenden Beschichtungen mit sich bringen kann. Die vorliegende Erfindung ist somit nicht auf eine beliebige Reihenfolge der Zugabe bzw. Umsetzung der einzelnen Bestandteile der Polyurethanbeschichtung beschränkt.

Dann wird weiteres Lösungsmittel zugesetzt und das gegebenenfalls gelöste Kettenverlängerungsdiamin bzw. der gelöste Kettenverlängerungsaminoalkohol (Verbindung (c)) zugegeben.

Die weitere Zugabe des Lösemittel erfolgt vorzugsweise schrittweise, um die Reaktion nicht unnötig zu verlangsamen, was bei einer vollständigen Zugabe der Lösemittelmenge beispielsweise am Anfang der Umsetzung passieren würde. Ferner ist man bei einem hohen Gehalt an Lösemittel zum Beginn der Reaktion an eine im Verhältnis niedrige Temperatur gebunden, welche von der Art des Lösemittels zumindest mitbestimmt wird. Auch dieses führt zu einer Verlangsamung der Reaktion.

Nach Erreichen der Zielviskosität können die noch verbleibenden Reste an NCO durch ein monofunktionelles aliphatisches Amin blockiert werden. Bevorzugt blockiert man die noch verbliebenen Isocanatgruppen durch Umsetzung mit den im Lösungsmittelgemisch enthaltenen Alkoholen.

Als Lösungsmittel für die Herstellung und die Anwendung der erfindungsgemäßen Polyurethanharnstoff-Lösungen kommen alle denkbaren Lösungsmittel und Lösungsmittelgemische wie Dimethylformamid, N-Methylacetamid, Tetramethylharnstoff, N-Methylpyrrolidon, aromatische Lösungsmittel wie Toluol, lineare und cyclische Ester, Ether, Ketone und Alkohole in Frage. Beispiele für Ester und Ketone sind beispielsweise Ethylacetat, Butylacetat, Aceton, γ-Butyrolacton, Methylethylketon und Methylisobutylketon.

Bevorzugt sind Mischungen aus Alkoholen mit Toluol. Beispiele für die Alkohole, die gemeinsam mit dem Toluol verwendet werden, sind Ethanol, n-Propanol, iso-Propanol und 1-Methoxy-2-propanol.

Im Allgemeinen wird in der Umsetzung so viel Lösungsmittel eingesetzt, dass man ungefähr 10 bis 50 gew.-%ige Lösungen, besonders bevorzugt ungefähr 15 bis 45 gew.-%ige Lösungen, besonders bevorzugt ungefähr 20 bis 40 gew.-%ige Lösungen, erhält.

Der Feststoffgehalt der Polyurethanharnstofflösungen liegt im Allgemeinen zwischen 5 bis 60 Gew.-%, vorzugsweise 10 bis 40 Gew.-%. Für Beschichtungsversuche können die Polyurethanharnstofflösungen beliebig mit Toluol/Alkohol-Gemischen verdünnt werden, um die Dicke der Beschichtung variabel einstellen zu können. Alle Konzentrationen von 1 bis 60 Gew.-% sind möglich, bevorzugt sind Konzentrationen im Bereich 1 bis 40 Gew.-%.

Dabei können beliebige Schichtdicken erreicht werden wie beispielsweise einige 100 nm bis hinauf zu einigen 100 µm, wobei im Rahmen der vorliegenden Erfindung auch höhere und geringere Dicken möglich sind.

Die Herstellung der erfindungsgemäßen Polyurthanharnstoff-Lösungen, welche den silberhaltigen Bestandteil umfassen, erfolgt durch Zugabe des mindestens einen silberhaltigen Bestandteils in fester oder dispergierter Form zu der Polyurethan-Polyharnstofflösung und anschließendes Homogenisieren durch Rühren oder Schütteln.

Die Partikel sind für die Zugabe zu organischen Polyurethanharnstofflösungen in organischen Lösungsmitteln dispergiert. Weitere Zusätze wie beispielsweise Antioxidantien oder Pigmente können ebenfalls verwendet werden. Darüber hinaus können gegebenenfalls noch weitere Zusätze wie Griffhilfsmittel, Farbstoffe, Mattierungsmittel, UV-Stabilisatoren, Lichtstabilisatoren, Hydrophobierungsmittel, Hydrophilierungsmittel und/oder Verlaufshilfsmittel verwendet werden.

Ausgehend von diesen Lösungen werden dann durch die zuvor beschriebenen Verfahren die erfindungsgemäß vorgesehenen Beschichtungen hergestellt.

Die Vorteile der erfindungsgemäßen Polyurethan-Lösungen werden durch Vergleichsversuche in den folgenden Beispielen dargelegt.

### Beispiele

Die Ermittlung des NCO-Gehaltes der in den Beispielen und Vergleichsbeispielen beschriebenen Harze erfolgte durch Titration gemäß DIN EN ISO 11909.

Die Bestimmung der Festkörpergehalte erfolgte nach DIN-EN ISO 3251. Es wurden 1 g Polyurethandispersion bei 115 °C bis zur Gewichtskonstanz (15-20 min) mittels eines Infrarottrockners getrocknet.

Die Messung der mittleren Teilchengrößen der Polyurethandispersionen erfolgt mit Hilfe des High Performance Particle Sizer (HPPS 3.3) der Firma Malvern Instruments.

Die in % angegebenen Mengenangaben verstehen sich, wenn nicht anders vermerkt, als Gew.-% und beziehen sich auf die erhaltene Gesamtlösung.

**Verwendete Substanzen und Abkürzungen:**

| | |
|---|---|
| Desmophen^{®} C2200: | Polycarbonatpolyol, OH-Zahl 56 mg KOH/g, zahlenmittleresMolekulargewicht 2000 g/mol (Bayer AG, Leverkusen, DE) |
| | |
| PolyTHF^{®} 1000: | Polytetramethylenglykolpolyol, OH-Zahl 110 mg KOH/g, zahlenmittleres Molekulargewicht 1000 g/mol (BASF AG, Ludwigshafen, DE) |
| | |
| PolyTHF^{®} 2000: | Polytetramethylenglykolpolyol, OH-Zahl 56 mg KOH/g, zahlenmitt-leres Molekulargewicht 2000 g/mol (BASF AG, Ludwigshafen, DE) |
| | |
| Polyether LB 25: | (monofunktioneller Polyether auf Ethylenoxid-/Propylenoxidbasis zahlenmittleres Molekulargewicht 2250 g/mol, OH-Zahl 25 mg KOH/g (Bayer AG, Leverkusen, DE) |

### Beispiel 1:

Dieses Beispiel beschreibt die Herstellung einer erfindungsgemäßen Polyurethanharnstoff-Lösung

197,4 g PolyTHF^{®} 2000, 15,0 g LB 25 und 47,8 g 4,4'-Bis(isocyanatocyclohexyl)methan (H₁₂MDI) wurden bei 110 °C bis zu einem konstanten NCO-Gehalt von 2,5 % umgesetzt. Man ließ abkühlen und verdünnte mit 350,0 g Toluol und 200 g iso-Propanol. Bei Raumtemperatur wurden eine Lösung von 11,7 g Isophorondiamin in 90,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 912 g einer 30,0%igen Polyurethanharnstofflösung in Toluol/iso-Propanol/1-Methoxypropanol-2 mit einer Viskosität von 26800 mPas bei 22 °C.

### Beispiel 2:

Dieses Beispiel beschreibt die Herstellung einer erfindungsgemäßen Polyurethanharnstoff-Lösung.

194,0 g PolyTHF^{®} 2000, 22,6 g LB 25 und 47,8 g 4,4'-Bis(isocyanatocyclohexyl)methan (H₁₂MDI) wurden bei 110 °C bis zu einem konstanten NCO-Gehalt von 2,3 % umgesetzt. Man ließ abkühlen und verdünnte mit 350,0 g Toluol und 200 g iso-Propanol. Bei Raumtemperatur wurden eine Lösung von 12,1 g Isophorondiamin in 89,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 916 g einer 30,7%igen Polyurethanharnstofflösung in Toluol/iso-Propanol/1-Methoxypropanol-2 mit einer Viskosität von 15200 mPas bei 22 °C.

### Beispiel 3:

Dieses Beispiel beschreibt die Herstellung einer erfindungsgemäßen Polyurethanharnstoff-Lösung.

190,6 g PolyTHF^{®} 2000, 30,0 g LB 25 und 47,8 g 4,4'-Bis(isocyanatocyclohexyl)methan (H₁₂MDI) wurden bei 110 °C bis zu einem konstanten NCO-Gehalt von 2,3 % umgesetzt. Man ließ abkühlen und verdünnte mit 350,0 g Toluol und 200 g iso-Propanol. Bei Raumtemperatur wurden eine Lösung von 11,6 g Isophorondiamin in 89,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 919 g einer 30,7%igen Polyurethanharnstofflösung in Toluol/iso-Propanol/1-Methoxypropanol-2 mit einer Viskosität von 21000 mPas bei 22 °C.

### Beispiel 4:

Dieses Beispiel beschreibt die Herstellung einer erfindungsgemäßen Polyurethanharnstoff-Lösung.

202,2 g Desmophen^{®} C2200, 15,0 g LB 25 und 47,8 g 4,4'-Bis(isocyanatocyclohexyl)methan (H₁₂MDI) wurden bei 110 °C bis zu einem konstanten NCO-Gehalt von 2,5 % umgesetzt.

Man ließ abkühlen und verdünnte mit 350,0 g Toluol und 200 g iso-Propanol. Bei Raumtemperatur wurden eine Lösung von 12,4 g Isophorondiamin in 94,0 g 1-Methoxypropanol-2 zugegeben. Nach beendetem Aufbau des Molgewichtes und Erreichen des gewünschten Viskositätsbereiches rührte man weitere 3,5 Stunden, um den restlichen Isocyanatgehalt mit iso-Propanol zu blockieren. Man erhielt 921 g einer 30,0%igen Polyurethanharnstofflösung in Toluol/iso-Propanol/1-Methoxypropanol-2 mit einer Viskosität von 34600 mPas bei 22 °C.

### Beispiel 5:

Dieses Beispiel beschreibt die Herstellung einer erfindungsgemäßen Polyurethanharnstoff-Lösung.

198,6 g Desmophen^{®} C 2200, 23,0 g LB 25 und 47,8 g 4,4'-Bis(isocyanatocyclohexyl)methan (H₁₂MDI) wurden bei 110 °C bis zu einem konstanten NCO-Gehalt von 2,4 % umgesetzt. Man ließ abkühlen und verdünnte mit 350,0 g Toluol und 200 g iso-Propanol. Bei Raumtemperatur wurden eine Lösung von 12,5 g Isophorondiamin in 95,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 927 g einer 30,4%igen Polyurethanharnstofflösung in Toluol/iso-Propanol/1-Methoxypropanol-2 mit einer Viskosität von 19600 mPas bei 22 °C.

### Beispiel 6:

Dieses Beispiel beschreibt die Herstellung einer erfindungsgemäßen Polyurethanharnstofflösung.

195,4 g Desmophen^{®} C 2200, 30,0 g LB 25 und 47,8 g 4,4'-Bis(isocyanatocyclohexyl)methan (H₁₂MDI) wurden bei 110 °C bis zu einem konstanten NCO-Gehalt von 2,3 % umgesetzt. Man ließ abkühlen und verdünnte mit 350,0 g Toluol und 200 g iso-Propanol. Bei Raumtemperatur wurden eine Lösung von 12,7 g Isophorondiamin in 94,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 930 g einer 30,7%igen Polyurethanharnstofflösung in Toluol/iso-Propanol/1-Methoxypropanol-2 mit einer Viskosität von 38600 mPas bei 22 °C.

### Beispiel 7:

Dieses Beispiel beschreibt die Herstellung einer erfindungsgemäßen Polyurethanharnstofflösung.

215,0 g PolyTHF^{®} 2000 und 47,8 g 4,4'-Bis(isocyanatocyclohexyl)methan (H₁₂MDI) wurden bei 110 °C bis zu einem konstanten NCO-Gehalt von 2,4 % umgesetzt. Man ließ abkühlen und verdünnte mit 350,0 g Toluol und 200 g iso-Propanol. Bei Raumtemperatur wurden eine Lösung von 10,0 g Isophorondiamin in 82,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 905 g einer 30,2%igen Polyurethanharnstofflösung in Toluol/iso-Propanol/1-Methoxypropanol-2 mit einer Viskosität von 19800 mPas bei 22 °C.

### Beispiel 8:

Dieses Beispiel beschreibt die Herstellung einer erfindungsgemäßen Polyurethanharnstofflösung.

107,0 g PolyTHF^{®} 1000 und 47,8 g 4,4'-Bis(isocyanatocyclohexyl)methan (H₁₂MDI) wurden bei 110 °C bis zu einem konstanten NCO-Gehalt von 3,9 % umgesetzt. Man ließ abkühlen und verdünnte mit 350,0 g Toluol und 200 g iso-Propanol. Bei Raumtemperatur wurden eine Lösung von 10,9 g Isophorondiamin in 94 g 1-Methoxypropanol-2 zugegeben. Nach beendetem Aufbau des Molgewichtes und Erreichen des gewünschten Viskositätsbereiches rührte man weitere 1,5 Stunden, um den restlichen Isocyanatgehalt mit iso-Propanol zu blockieren. Man erhielt 810 g einer 20,5%igen Polyurethanharnstofflösung in Toluol/iso-Propanol/l-Methoxypropanol-2 mit einer Viskosität von 40000 mPas bei 22 °C.

### Beispiel 9:

Dieses Beispiel beschreibt die Herstellung einer erfindungsgemäßen Polyurethanharnstofflösung.

219,0 g Desmophen^{®} C 2200 und 47,8 g 4,4'-Bis(isocyanatocyclohexyl)methan (H₁₂MDI) wurden bei 110 °C bis zu einem konstanten NCO-Gehalt von 2,4 % umgesetzt. Man ließ abkühlen und verdünnte mit 350,0 g Toluol und 200 g iso-Propanol. Bei Raumtemperatur wurden eine Lösung von 10,7 g Isophorondiamin in 90,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 918 g einer 31,1%igen Polyurethanharnstofflösung in Toluol/iso-Propanol/1-Methoxypropanol-2 mit einer Viskosität von 19400 mPas bei 22 °C.

### Beispiel 10: Silberhaltige Polyurethanlösungen

Es wurden eine 0,054 molare Silbernitratiösung mit einer Mischung aus einer 0,054 molaren Natronlauge und dem Dispergierhilfsmittel Disperbyk 190 (Hersteller BYK Chemie) (1 g/l) in einem Volumenverhältnis von 1:1 versetzt und 10 min gerührt. Ein braun gefärbtes Ag₂O-Nanosol entstand. Zu dieser Reaktionsmischung wurde unter Rühren eine wässrige 4,6 molare Formaldehyd-Lösung zugesetzt, so dass das molare Verhältnis Ag⁺ zu Reduktionsmittel 1:10 betrug. Diese Mischung wurde auf 60°C erwärmt, 30 min bei dieser Temperatur gehalten und anschließend abgekühlt. Die Partikel wurden mittels Zentrifugation (60 min bei 30000 U/min) aufgereinigt und durch Eintrag von Ultraschall (1 min) in vollentsalztem Wasser redispergiert. Dieser Vorgang wurde zweimal wiederholt. Ein kolloidal-stabiles Sol mit einem Feststoffgehalt von 5 Gew.-% (Silberpartikel und Dispergierhilsmittel) wurde so erhalten. Die Ausbeute beträgt knapp 100 %. Die Silberdispersion enthält nach der Zentrifugation laut Elementaranalyse 3 Gew.-% Disperbyk 190 bezogen auf den Silbergehalt. Eine Untersuchung mittels Laserkorrelationsspektroskopie ergab einen effektiven Durchmesser der Partikel von 73 nm.

Für die Zugabe des Silbersols in die erfindungsgemäßen Polyurethanharnstofflösungen muss das erhaltene Silber erst in ein organisches Medium redispergiert werden. Hierzu wird das wässrige Silber-Sol fast zur Trockene im Rotationsverdampfer einrotiert. Anschließend wird das Silberpulver in einem Toluol/Isopropanol(2.1)-Gemisch aufgenommen und mit einem Ultraschallfinger wenige Sekunden redispergiert.

50 ml der Polyurethanlösungen der Beispiele 1 bis 9 wurden mit einer 15%igen kolloidalen Silberdispersion, dessen Herstellung vorstehend beschrieben ist, versetzt und durch Schütteln homogenisiert. In die Polyurethanlösungen der Beispiele 1 bis 9 wird so viel Silberdispersion zugegeben, dass die Dispersionen bezogen auf den festen Polymergehalt 1 Gew.-% Silber enthalten.

**Tabelle 1: Polyurethanlösungen mit 1 Gew.-% nanokristallinem Silber**

| Beispiel | Produkt |
|---|---|
| 10a | PU-Lösungen des Beispiels 1 mit 1 Gew.-% nanokristallinem Silber |
| 10b | PU-Lösungen des Beispiels 2 mit 1 Gew.-% nanokristallinem Silber |
| 10c | PU-Lösungen des Beispiels 3 mit 1 Gew.-% nanokristallinem Silber |
| 10d | PU-Lösungen des Beispiels 4 mit 1 Gew.-% nanokristallinem Silber |
| 10e | PU-Lösungen des Beispiels 5 mit 1 Gew.-% nanokristallinem Silber |
| 10f | PU-Lösungen des Beispiels 6 mit 1 Gew.-% nanokristallinem Silber |
| 10g | PU-Lösungen des Beispiels 7 mit 1 Gew.-% nanokristallinem Silber |
| 10h | PU-Lösungen des Beispiels 8 mit 1 Gew.-% nanokristallinem Silber |
| 10i | PU-Lösungen des Beispiels 9 mit 1 Gew.-% nanokristallinem Silber |

### Beispiel 11: Ag-Freisetzungsstudie chemisch

Die silberhaltigen Beschichtungen zur Messung der Silberfreisetzung wurden auf Plättchen aus Polyurethan (Thermoplastisches Polyurethan Texin 3041, Bayer MaterialScience AG) der Größe 25x75 mm mit Hilfe eines Spincoaters (RC5 Gyrset 5, Karl Süss, Garching, Deutschland) hergestellt. Die Plättchen wurde hierzu auf dem Probenteller des Spincoaters eingespannt und mit ca. 2,5 - 3 g der Polyurethanlösungen homogen bedeckt. Die verwendeten Polyurethanlösungen der Beispiele 1-9 wurden hierzu mit einem Gemisch aus Toluol und Isopropanol (65Gew.-% / 35 Gew.-%) auf die Hälfte der ursprünglichen Konzentration verdünnt. Durch Rotation des Probentellers für 20 sec bei 1300 Umdrehungen pro Minute erhielt man homogene Beschichtungen, die 24 h bei 50 °C getrocknet wurden. Von den so erhaltenen beschichteten Plättchen wurden Stücke von etwa 4 cm² hergestellt und zur Messung der freigesetzten Silbermengen herangezogen.

Die verschiedenen silberhaltigen Polyurethanbeschichtungen der Beispiele 10a bis 10i wurden in Tablettenröhrchen mit 2,5 ml destilliertem Wasser überschichtet und 1 Woche im Brutschrank bei 37 °C gelagert. Das Wasser wurde abgenommen und die vom Film in die Flüssigkeit abgegebene Silbermenge per Atomabsorptionsspektroskopie bestimmt. Die trockenen Filme auf den Polyurethanplatten wurden wieder mit 2,5 ml Wasser überschichtet und weiter bei 37 °C gelagert. Das ganze Verfahren wurde 5 mal wiederholt, so dass Silberfreisetzungen für mehrere Wochen bestimmt werden können.

**Tabelle 2: Silberfreisetzung der Filme in wässrige Umgebung**

| | Beispiel 10a | Beispiel 10b | Beispiel 10c | Beispiel 10d | Beispiel 10e | Beispiel 10f | Beispiel 10g | Beispiel 10h | Beispiel 10i |
|---|---|---|---|---|---|---|---|---|---|
| ng Ag (Woche 1) | 70 | 128 | 145 | 148 | 53 | 70 | 25 | 58 | 50 |
| ng Ag (Woche 2) | 33 | 70 | 55 | 13 | 8 | 5 | 23 | 38 | 15 |
| ng Ag (Woche 3) | 58 | 163 | 113 | 15 | 83 | 40 | 68 | 115 | 25 |
| ng Ag (Woche 4) | 15 | 28 | 18 | 5 | 5 | 5 | 3 | 15 | 5 |
| ng Ag (Woche 5) | 15 | 28 | 30 | 8 | 5 | 13 | - | 20 | 5 |

Die Ergebnisse zeigen, dass die Beschichtungen über einen längeren Zeitraum Silber abgeben. Höhere Silberfreisetzungen ergeben die mit dem Polyether LB 25 ausgerüsteten Beschichtungen 10a bis 10f. Über einen Zeitraum von 5 Wochen geben die mit PolyTHF^{®} 2000 und LB 25 hergestellten Filme 10b und 10c die etwas höheren Werte an Silber frei als die mit Polycarbonat hergestellten Filme 10d bis 10e.

### Beispiel 12: Antimikrobielle Aktivität von silberhaltigen Beschichtungen

Polyurethanplättchen aus den silberhaltigen Polyurethandispersionen der Beispiele 10a bis 10i wurden in einer Bakteriensuspension von *Escherichia coli* ATCC 25922 auf ihre abtötende Wirkung hin untersucht.

Der Testkeim *E. coli* ATCC 25922 wurde in einer Übernachtkultur auf Columbia Agar (Columbian Blutagar Platten, Firma Becton Dickinson, # 254071) bei 37°C kultiviert. Anschließend wurden einige Kolonien in PBS (PBS pH 7,2, Firma Gibco, #20012) mit 5 % Müller Hinton Medium (Becton Dickinson, #257092) aufgeschwemmt und eine Zellzahl von ca. 1 x 10⁵ Keime/ml eingestellt. Jeweils 100 µl dieser Suspension wurden mit Hilfe eines 20 x 20 mm großen Parafilms auf dem Testmaterial verteilt, so dass die Fläche gleichmäßig mit Zellsuspension benetzt war. Anschließend wurde das Testmaterial mit der Bakteriensuspension 6 h bei 37 °C in einer Feuchtkammer inkubiert. Nach 6 h wurden 20 µl der Zellsuspension zur Wachstumskontrolle entnommen. Die Zellzahl wurde durch Reihenverdünnung und Ausplattieren der Verdünnungsstufen auf Agarplatten bestimmt. Dabei wurden nur lebende Zellen ermittelt. Die Zellzahl wurde als Colonie forming units (CFU)/ ml angegeben. Anschließend wurde der Parafilm vom Testmaterial entnommen und das Testmaterial 3-mal mit jeweils 4 ml PBS gewaschen um frei-schwimmende Zellen zu entfernen.

Für die Bestimmung der antiadhäsiven Aktivität wurden die beschichteten Plättchen dreimal mit 4 ml PBS gewaschen, um nicht adhärierte Zellen zu entfernen. Danach wurden die beschichteten Plättchen in 15 ml PBS überführt und zwei Minuten im Ultraschallbad beschallt, um die anheftenden Zellen abzulösen. Von der PBS Lösung, die die abgelösten Zellen enthält, wurde ebenfalls eine Zellzahlbestimmung über Verdünnungsreihen und Ausplattierung auf Agarplatten durchgeführt. Auch hierbei wurden nur lebende Zellen erfasst. Falls genügend Testmaterial zur Verfügung stand, wurde dieser Test jeweils dreimal unabhängig voneinander durchgeführt. Das Ergebnis wurde als Mittelwert der CFU/ml mit Standardabweichung angegeben.

**Tabelle 3: Antibakterielle Wirkung der Beschichtungen der Beispiele 10 a bis 10 i**

| Beschichtung | Keimwachstum (CFU/ml) | Zelladhäsion (CFU/ml) |
|---|---|---|
| Beispiel 10 a | < 10³ | < 10² |
| Beispiel 10 b | 8 x 10³ | < 10² |
| Beispiel 10 c | < 10³ | < 10² |
| Beispiel 10 d | < 10³ | < 10² |
| Beispiel 10 e | < 10³ | < 10² |
| Beispiel 10 f | 1 x 10⁴ | < 10² |
| Beispiel 10 g | < 10³ | < 10² |
| Beispiel 10 h | 1 x 10⁸ | < 10² |
| Beispiel 10 i | 1 x 10⁷ | 5 x 10² |
| Negativkontrolle (Glasobjektträger) | 1 x 10⁸ | 5 x 10⁵ |

Die Tabelle verdeutlicht sehr klar die antibakterielle Wirksamkeit der untersuchten silberhaltigen Beschichtungen. Bis auf ein Material zeigen alle anderen Beschichtungen keine signifikanten adhärierten Zellpopulationen auf der Beschichtung, was für die Bildung eines Biofilms sehr wichtig ist. Darüber hinaus wird bei den meisten Beschichtungen auch das Keimwachstum in der umgebenen Bakteriensuspension deutlich unterdrückt.

## Patentansprüche

1. Lösung eines nichtionischen Polyurethanharnstoffes, welche als antimikrobiellen Wirkstoff einen silberhaltigen Bestandteil aufweist.

2. Lösung nach Anspruch 1, **dadurch gekennzeichnet, dass** der Polyurethanharnstoff mit einer Copolymereinheit aus Polyethylenoxid und Polypropylenoxid terminiert ist.

3. Lösung nach Anspruch 1 oder 2, **dadurch gekennzeichnet, dass** die Polyurethanharnstoff zumindest aufgebaut wird aus den folgenden Aufbaukomponenten:
a) mindestens einem Makropolyol;
b) mindestens einem Polyisocyanat;
c) mindestens einem Diamin oder einem Aminoalkohol;
d) mindestens einem monofunktionellen Polyoxyalkylenether mit einer Copolymereinheit aus Polyethylenoxid und Polypropylenoxid terminiert ist; und
h) antimikrobiell wirksames Silber.

4. Lösung nach einem der Ansprüche 1 bis 3, **dadurch gekennzeichnet, dass** der silberhaltige Bestandteil ausgewählt ist aus der Gruppe, bestehend aus einem hochporösen Silberpulver, Silber auf Trägermaterialien oder kolloidalen Silber-Solen.

5. Lösung nach einem der Ansprüche 1 bis 4, **dadurch gekennzeichnet, dass** zur antimikoribiellen Ausrüstung die Lösung nanokristalline Silberteilchen mit einer mittleren Größe von 1 bis 1000 nm umfasst.

6. Lösung nach einem der Ansprüche 1 bis 5, **dadurch gekennzeichnet, dass** die Menge an Silber, bezogen auf die Menge an festem Polymer und gerechnet als Ag und Ag⁺, 0,1 bis 10 Gew.-% beträgt.

7. Verfahren zur Herstellung einer Lösung eines nichtionisch stabilisierten Polyurethanharnstoffes gemäß einem der Ansprüche 1 bis 6, **dadurch gekennzeichnet, dass** zur Herstellung
(a) das mindestens eine Makropolyol, das mindestens eine Polyisocyanat, gegebenenfalls der mindestens eine monofunktionelle Polyoxyalkylether und gegebenenfalls das mindestens eine Polyol in der Schmelze oder in Gegenwart eines Lösemittels in Lösung miteinander umgesetzt werden, bis alle Hydroxylgruppen verbraucht sind;
(b) weitere Lösemittel zugesetzt und das gegebenenfalls gelöste Diamin oder der gegebenenfalls gelöste Aminoalkohol zugegeben wird, und
(c) gegebenenfalls nach Erreichen der Zielviskosität noch verbleibenden Reste an NCO-Gruppen durch ein monofunktionelles aliphatisches Amin blockiert werden.

8. Verfahren nach Anspruch 7, **dadurch gekennzeichnet, dass** nach der Herstellung der Polyurethanharnstoff-Lösung die resultierende Lösung mit einem silberhaltigen Bestandteil versetzt wird.

9. Verfahren nach Anspruch 7 oder 8, **dadurch gekennzeichnet, dass** das Lösemittel ausgewählt ist aus der Gruppe, bestehend aus Dimethylformamid, N-Methylacetamid, Tetramethylharnstoff, N-Methylpyrrolidon, γ-Butyrolacton, aromatischen Lösungsmitteln, linearen und cyclischen Estern, Ethern, Ketonen, Alkoholen und Mischungen davon.

10. Verfahren nach einem der Ansprüche 7 bis 9, **dadurch gekennzeichnet, dass** der Feststoffgehalt der Polyurethanlösung zwischen 5 bis 60 Gew.-% beträgt.

11. Polyurethanharnstoff-Lösung, erhältlich nach einem Verfahren gemäß einem der Ansprüche 6 bis 9.

12. Verwendung der Polyurethanharnstoff-Lösung gemäß einem der Ansprüche 1 bis 6 oder 11 zur Herstellung von Beschichtungen.
